(19) Europäisches Patentamt
European Patent Office
Office européen des brevets

(11) Veröffentlichungsnummer: **0 687 469 A2**

# (12) EUROPÄISCHE PATENTANMELDUNG

(21) Anmeldenummer: **95108959.8**

(51) Int. Cl.⁶: **A61K 31/445**

(22) Anmeldetag: **10.06.95**

(30) Priorität: **17.06.94 DE 4421324**

(43) Veröffentlichungstag der Anmeldung: **20.12.95 Patentblatt 95/51**

(84) Benannte Vertragsstaaten: **AT BE CH DE DK ES FR GB GR IE IT LI LU NL PT SE**

(71) Anmelder: **Kali-Chemie Pharma GmbH**
**Hans-Böckler-Allee 20**
**D-30173 Hannover (DE)**

(72) Erfinder: **Ziegler, Dieter, Dr.**
**Müggenwinkel 44**
**D-30966 Hemmingen (DE)**
Erfinder: **Brückner, Reinhard, Dr.**
**Homburgweg 8**
**D-30559 Hannover (DE)**
Erfinder: **Schön, Uwe, Dr.**
**Föhrenkamp 12**
**D-31303 Burgdorf (DE)**

(74) Vertreter: **Lauer, Dieter, Dr.**
**Solvay Pharma Deutschland GmbH**
**Hans-Böckler-Allee 20**
**D-30173 Hannover (DE)**

## (54) Anti-arrhythmische 3,7-Diazabicyclo(3,3,1)nonan-Verbindungen

(57) Es wird die Verwendung von N-Isobutyl-N'-isopropyl-9,9-pentamethylen-3,7-diazabicyclo[3,3,1]nonan und dessen physiologisch verträglichen Säureadditionssalzen zur Herstellung von pharmazeutischen Zubereitungen zur Behandlung von supraventrikulären Arrythmien beschrieben.

EP 0 687 469 A2

Die vorliegende Erfindung betrifft die Verwendung von N-Isobutyl-N'-isopropyl-9,9-pentamethylen-3,7-diazabicyclo[3,3,1]nonan (= Bertosamil) und dessen physiologisch verträglichen Säureadditionssalzen zur Behandlung von supraventrikulären Arrthymien, und zur Herstellung von für diese Behandlung geeigneten Arzneimitteln.

Der Erfindung liegt die Aufgabe zugrunde, neue pharmazeutische Zubereitungen zur Behandlung von Herzrhythmusstörungen, welche ihren Ursprung im Herzvorhof haben, zu entwickeln.

Erfindungsgemäß werden zur Herstellung von pharmazeutischen Zubereitungen zur Behandlung von supraventrikulären Arrythmien N-Isobutyl-N'-isopropyl-9,9-pentamethylen-3,7-diazabicyclo[3,3,1]nonan der Formel I

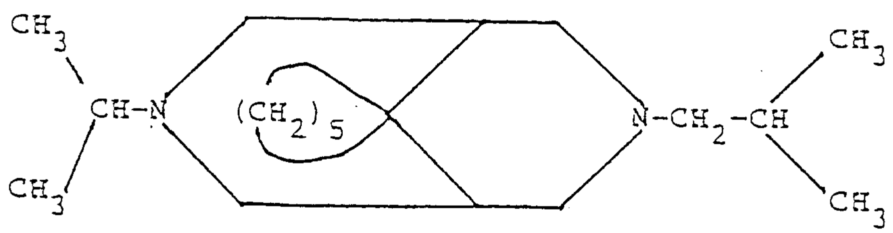

I

und dessen physiologisch verträgliche Säureadditionssalze verwendet.

Als physiologisch verträgliche Säureadditionssalze des Bertosamils eignen sich Salze mit anorganischen Säuren z. B. Halogenwasserstoffsäuren, oder mit organischen Säuren, beispielsweise niederen aliphatischen Mono- oder Dicarbonsäuren wie Essigsäure, Fumarsäure oder Weinsäure oder aromatischen Carbonsäuren wie z. B. Salicylsäure.

Die erfindungsgemäß zur Behandlung von supraventrikulären Arrythmien eingesetzten Verbindungen fallen unter den Umfang von in der europäischen Patentschrift Nr. 0 103 833 beschriebenen 9,9,N,N'-tetrasubstituierten 3,7-Diazabicyclo[3,3,1]nonan-Verbindungen mit den Sauerstoffverbrauch des Herzens einsparenden, die Herzfrequenz beeinflussenden und herzrhythmisierenden Wirkungen, und sind aus dieser Patentschrift bekannt. Die Verbindungen können auf an sich bekannte Weise nach den in der vorgenannten europäischen Patentschrift beschriebenen Verfahren oder analog zu diesen Verfahren hergestellt werden.

Aus der vorgenannten europäischen Patentschrift ist bekannt, daß die Verbindungen eine antiarrhythmische Wirkung mit einem günstigen Verhältnis von antiarrhythmischen, bzw. die Refraktärzeit des Herzens verlängernden Wirkungen zu negativ inotropen Nebenwirkungen aufweisen. Die rhythmischen Kontraktionen des Herzmuskels hängen wesentlich von der Wiedererregbarkeit (= Refraktärität) und der Dauer der elektrischen Erregung (= Depolarisation) des Herzmuskelsgewebes ab. Die Wirkung chemischer Substanzen auf den Herzrhythmus wird durch Messung elektrophysiologischer Parameter, z. B. der Refraktärzeit und der Dauer der Depolarisation (= Aktionspotentialdauer), geprüft. Die Refraktärität des Herzens wird vorwiegend von von Kalium-Ionen getragenen Strömen durch die Zellmembran bestimmt. Hierbei spielen insbesondere der transiente Kaliumauswärtsstrom (= Ito) und der verzögerte Kaliumauswärtsstrom (= Ik) eine Rolle. Der Ito ist ein schnell aktivierender, zeitabhängig inaktivierender von Kaliumionen getragener, auswärtsgerichteter Ionenstrom. Er ist der repolarisierende Strom in der frühen Phase und ist daher in Geweben mit früher Repolarisation, z. B. dem Vorhofsgewebe, von physiologischer Bedeutung. Der Ik ist ein langsam aktivierender, nicht zeitabhängig inaktivierender, von Kalimionen getragener, auswärtsgerichteter Ionenstrom. Er ist der repolarisierende Strom in der späten Phase und ist daher in Geweben mit später Repolarisation, z. B. Ventrikelgewebe, von physiologischer Bedeutung. Eine Blockade dieser Ströme führt zu einer Verlängerung der Refraktärzeit und der Depolarisationsdauer, in der das Herz für unerwünschte Extraerregungen unempfindlich ist. Pharmakologisch aktive Substanzen können durch Hemmung oder Blockade dieser Ströme eine antiarrhythmische Wirkung ausüben.

Es wurde nun überraschenderweise gefunden, daß Bertosamil und seine Säureadditionssalze eine die Refraktärzeit und die Depolarisationsdauer verlängernde Wirkung selektiv am Vorhof des Herzens aufweisen. Aus Untersuchungen der Wirkungen von Bertosamil und seinen Säureadditionssalzen auf Refraktärität und Depolarisation an isolierten Herzmuskelpräparaten von Herzvorhof und Herzkammer von Meerschweinchen wurde gefunden, daß der deutlich verlängernden Wirkung am Vorhof keine oder eine geringfügig verkürzende Wirkung an der Herzkammer gegenübersteht. Eine Untersuchung der Wirkung von Bertosamil und seinen Säureadditionssalzen auf die Kaliumauswärtsströme Ito und Ik bestätigte die Selektivität zugunsten einer Blockade des transienten Auswärtsstroms Ito.

Die für Bertosamil gefundene selektive Verzögerung der Wiedererregbarkeit am Herzvorhof, d. h. die besonders im Herzvorhof ausgeprägte antiarrhythmische Wirkung, macht die Verbindung geeignet zur Behandlung von Arrythmien, die ihren Ursprung im Herzvorhof haben. Damit bietet sich eine bisher unerwartete Möglichkeit zur Behandlung von supraventrikulären Arrythmien.

Die selektiv auf den Herzvorhof und den transienten Kaliumauswärtsstrom gerichteten antiarrhythmischen Wirkungen des Bertosamil und seiner Säureadditionssalze lassen sich in pharmakologischen Standardstests in vitro nachweisen.

Beschreibung der pharmakologischen Versuchsmethoden.

I. Messung der Wirkung der Substanz auf Refraktärzeit und Aktionspotentialdauer.

Die Experimente wurden an isolierten Herzmuskelgeweben aus Vorhof und Kammer von Meerschweinchen durchgeführt. Die Gewebestücke wurden in einer mit Sauerstoff durchperlten gewärmten Nährlösung befestigt und mittels zwei metallischer Reizelektroden kontinuierlich durch elektrische Impulse (2Hz, 0,5ms Dauer) zu Kontraktionen angeregt. Die Kontraktionen wurden mittels eines Schreibers registriert.

Die funktionelle Refraktärzeit ist das kürzeste Intervall zwischen einem doppelten elektrischen Impuls, der von einer doppelten Kontraktion beantwortet wird. Die Refraktärzeit wurde am Vorhofs- und Kammergewebe getrennt durch Variation des Intervalls zwischen dem konstanten (treibenden) Impuls und einem zusätzlichen Testimpuls bestimmt. Zur Prüfung der Wirkung der Testsubstanz wurde die Testsubstanz in einer Nährlösung zugegeben und es wurden die Refraktärzeiten vor und nach Zugabe der Testsubstanz miteinander verglichen.

Zur Messung der Aktionspotentialdauer wurde jeweils eine Mikroelektrode (in Form einer feinen mit 3-molarer KCl-Lösung gefüllten Glaskapillare) in eine Zelle des Gewebepräparates eingestochen, und es wurde die elektrische Aktivität bei jeder Kontraktion (= Aktionspotential) registriert. Die Dauer der Aktionspotentiale, d. h. die Dauer der elektrischen Erregung (= Depolarisation) wurde vor und nach Zugabe der Testsubstanz in die Nährlösung ermittelt.

Die mit Bertosamil bei einer Konzentration von 5 µ mol/l erzielten Ergebnisse sind in der nachfolgenden Tabelle 1 wiedergegeben.

**Tabelle 1**

| Testsubstanz, Dosis | Wirkung | |
|---|---|---|
| | funktionelle Refraktärzeit Verlängerung in ms | |
| | Herzvorhof | Herzkammer |
| Bertosamil, 5 µmol/l (als Sesquihydrogenfumarat-salz) | + 33 | - 3 |
| | Aktionspotentialdauer Verlängerung in ms | |
| | Herzvorhof | Herzkammer |
| Bertosamil, 5 µmol/l (als Dihydrogenfumaratsalz) | + 56 | - 9 |

B. Messung von Ionenströmen.

Die Experimente wurden mit der Spannungsklemmmethode an isolierten Herzmuskelzellen von Meerschweinchen und Ratten durchgeführt. Hierbei wurden die Messungen des Ito an Zellen von Rattenherzen und die Messungen des Ik an Zellen von Meerschweinchenherzen durchgeführt. Die Herzen wurden mit proteolytischen Enzymen (Collagenase) perfundiert, um das Bindegewebe zu lockern. Anschließend wurde das Gewebe mechanisch zerkleinert und sodann erfolgte eine weitere Vereinzelung der Zellen in einer Nährlösung im Schüttelbad. Durch anschließendes Zentrifugieren wurde eine Suspension von Herzmuskelzellen erhalten. Bei der angewendeten Spannungsklemmethode zur Messung der Membranströme wird mittels einer Mikroelektrode (in Form einer mit Elektrolytlösung gefüllten Glaskapillare) das Zellinnere mit einem Verstärker verbunden, der die Membranspannung vorgeben kann. Fließt nun ein Ionenstrom durch die Membran (= Öffnung des Ionenkanalproteins) und verschiebt die Membranspannung, kompensiert der Verstärker diese Verschiebung durch einen Gegenstrom, d. h. die Spannung ist sozusagen festgeklemmt. Der gemessene Gegenstrom entspricht in seinem zeitlichen Verlauf dem physiologischen Ionenstrom mit umgekehrtem Vorzeichen. Die Aktivierung der verschiedenen Ionenströme in der Zellmembran hängt charakteristisch von Spannung und Zeit ab. Daher ist es möglich durch Wahl geeigneter Spannungsprotokolle die verschiedenen Ströme zu differenzieren. Nach dieser Methode wurde der Einfluß einer Zugabe von 1 µmol/l Testsubstanz auf die verschiedenen Ströme erfaßt. Die mit Bertosamil erhaltenen Ergebnisse sind in der nachfolgenden Tabelle 2 wiedergegeben.

Tabelle 2

| Testsubstanz, Dosis | Blockade von Ionenströmen in % des Vorwerts | |
|---|---|---|
| | transienter Auswärtsstrom Ito | verz.gleichr. Auswärtsstrom Ik |
| Bertosamil, 1 µmol/l (als Sesquihydrogenfumaratsalz) | - 38 | - 13 |

Die vorstehenden pharmakologischen Versuchsergebnisse zeigen, daß Bertosamil und seine Säureadditionssalze antiarrhythmische Wirkungen selektiv am Herzvorhof ausüben und deshalb für die Behandlung von supraventrikulären Arrythmien geeignet sind. Die zu verwendenden Dosen können individuell verschieden sein und variieren naturgemäß je nach Art des zu behandelnden Zustandes und der Applikationsform. Im allgemeinen eignen sich zur Applikation am Menschen und an größeren Säugetieren jedoch Arzneiformen mit einem Wirkstoffgehalt von 50 bis 150 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt oder auch Suppositorien. Diese Präparate können pharmazeutisch übliche anorganische und/oder organische Trägerstoffe wie z. B. Talkum, Milchzucker oder Stärke neben pharmazeutisch üblichen Hilfsmitteln, beispielsweise Gleitmitteln oder Tablettensprengmitteln, enthalten. Flüssige Präparate wie Lösungen, Suspensionen oder Emulsionen der Wirkstoffe können die üblichen Verdünnungsmittel wie Wasser, Öle und/oder Suspensionsmittel wie Polyäthylenglykole und dergleichen enthalten. Es können zusätzlich weitere Hilfsstoffe zugegeben werden wie z. B. Konservierungsmittel, Geschmackskorregenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden.

Die folgenden Beispiele sollen die Herstellung von die Verbindungen enthaltenden pharmazeutischen Zubereitungen näher erläutern, ohne jedoch den Umfang der Anmeldung zu begrenzen.

**Beispiel 1:** Tabletten

Zusammensetzung:

| | |
|---|---|
| Bertosamil-sesquihydrogenfumarat | 100 Teile |
| Maisstärke | 100 Teile |
| Maisstärke (für Kleister) | 20 Teile |
| Precirol ato$^R$ ( = partielles Glycerinpalmitatstearat) | 5 Teile |
| Aerosil 200$^R$ ( = hochdisperse Kieselsäure) | 1 Teile |
| Talkum | 3 Teile |
| Gesamt | 229 Teile |

Herstellungsvorschrift:

Der Wirkstoff wurde mit der gleichen Menge Maisstärke in einem Mischer vermischt. Die entstandene Mischung wurde mit einem Kleister aus Maisstärke in entmineralisiertem Wasser durchfeuchtet und granuliert. Das feuchte Granulat wurde durch ein 2 mm-Sieb passiert, bei 40° auf Horden getrocknet und anschließend durch ein 1 mm-Sieb (Frewitt-Maschine) passiert. Nach dem Vermischen des Granulates mit Precirol, Talkum und hochdisperser Kieselsäure wurden daraus Tabletten mit einem Gewicht von 229 mg gepreßt, so daß jede Tablette 100 mg Wirkstoff enthielt.

**Beispiel 2:** Kapseln

Zusammensetzung:

| | |
|---|---|
| Bertosamil-sesquihydrogenfumarat | 100 Teile |
| Avicel$^R$ (mikrokristalline Cellulose) | 80 Teile |
| Hydroxymethylpropylcellulose | 20 Teile |
| Talkum | 4 Teile |
| Magnesiumstearat | 1 Teile |
| Aerosil 200$^R$ | 6 Teile |
| Gesamt | 211 Teile |

Herstellungsvorschrift:

Der Wirkstoff wurde mit mikrokristalliner Cellulose in einem Mischer vermischt. Die entstandene Mischung wurde mit einer Lösung von Hydroxymethylpropylcellulose in entmineralisiertem Wasser durchfeuchtet und granuliert. Das feuchte Granulat wurde durch ein 1,6 mm-Sieb (Frewitt-Maschine) passiert, bei 40 °C auf Horden getrocknet und anschließend durch ein 1 mm-Sieb (Frewitt) passiert. Nach dem Vermischen des Granulates mit Talkum, Magnesiumstearat und hochdisperser Kieselsäure (Aerosil 200$^R$ der Fa. Degussa) wurden von dem Gemisch jeweils 211 mg mittels automatischer Kapselmaschine in Hartgelatinekapseln der Größe 1 abgefüllt, so daß jede Kapsel 100 mg Wirkstoff enthielt.

## Patentansprüche

1. Verwendung von N-Isobutyl-N'-isopropyl-9,9-pentamethylen-3,7-diazabicyclo[3,3,1]nonan der Formel I

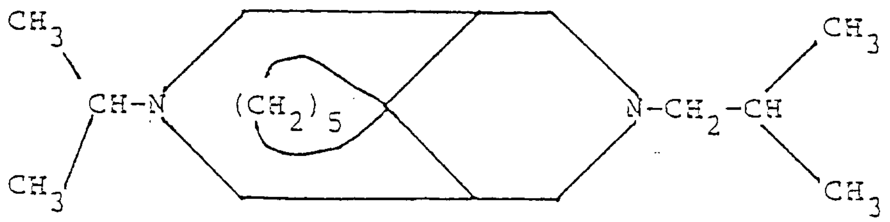

I

und dessen physiologisch verträglichen Säureadditionssalzen zur Herstellung von pharmazeutischen Zubereitungen zur Behandlung von supraventrikulären Arrythmien.

2. Verfahren zur Herstellung von pharmazeutischen Zubereitungen zur Behandlung von supraventrikulären Arrythmien, dadurch gekennzeichnet, daß man die Verbindung der Formel I gemäß Anspruch 1 oder deren physiologisch verträgliche Säureadditionssalze zusammen mit üblichen pharmazeutischen Hilfsstoffen in eine geeignete Arzneiform überführt.